(19) Europäisches Patentamt
European Patent Office
Office européen des brevets

(11) Veröffentlichungsnummer: **0 166 155**
**B1**

(12) # EUROPÄISCHE PATENTSCHRIFT

(45) Veröffentlichungstag der Patentschrift: **23.09.87**

(51) Int. Cl.⁴: **C 07 C 147/12,** A 61 K 7/13

(21) Anmeldenummer: **85105860.2**

(22) Anmeldetag: **13.05.85**

(54) **2-Alkylsulfonyl-1,4-diaminobenzole, Verfahren zu ihrer Herstellung sowie Oxidationshaarfärbemittel mit einem Gehalt an diesen Verbindungen.**

(30) Priorität: **29.06.84 DE 3423933**

(43) Veröffentlichungstag der Anmeldung: **02.01.86 Patentblatt 86/1**

(45) Bekanntmachung des Hinweises auf die Patenterteilung: **23.09.87 Patentblatt 87/39**

(84) Benannte Vertragsstaaten: **DE FR GB IT**

(56) Entgegenhaltungen:
**EP-A-0 007 537**
**US-A-3 118 943**

(73) Patentinhaber: **Wella Aktiengesellschaft, Berliner Allee 65, D-6100 Darmstadt (DE)**

(72) Erfinder: **Braun, Hans- Jürgen, Dr., Impasse de la Colline 3, CH- 1723 Marly (CH)**
Erfinder: **Konrad, Eugen, Mecklenburgerstrasse 101, D-6100 Darmstadt (DE)**
Erfinder: **Mager, Herbert, Dr., Beaumont 5, CH-1700 Fribourg (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 166 155 B1**

LIBER, STOCKHOLM 1987

## Beschreibung

Gegenstand der Erfindung sind Mittel zur oxidativen Färbung von Haaren auf der Basis von Entwickler- und Kupplersubstanzen, wobei als Kupplersubstanz neue 2-Alkylsulfonyl-1,4-diaminobenzole verwendet werden.

Für die Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden bevorzugt 2,5-Diamino-toluol, 4-Aminophenol und 1,4-Diaminobenzol verwendet, jedoch haben auch 2,5-Diaminoanisol, 2,5-Diaminobenzyl-alkohol und 2-(β-Hydroxyethyl)-1,4-diaminobenzol eine gewisse Bedeutung erlangt. In bestimmten Fällen kann auch Tetraaminopyrimidin als Entwicklersubstanz eingesetzt werden.

Zur Erzeugung von Naturtönen werden als Kupplersubstanzen vorwiegend Resorcin oder 4-Chlorresorcin in Verbindung mit m-Aminophenol verwendet. An Stelle von Resorcin können auch 4-Hydroxy-1,2-methylendioxybenzol oder 4-Amino-1,2-methylendioxybenzol eingesetzt werden. Zur Erzeugung von Blauanteilen in Schwarz- und Aschtönen kommen bevorzugt 2,4-Diaminophenolether wie 2,4-Diaminoanisol, 2,4-Diaminophenetol, 2,4-Diaminophenoxyethanol und 2-Amino-4-(2'-hydroxyethylamino)-anisol zum Einsatz. Ferner können bestimmte Pyridinderivate, wie zum Beispiel 3,5-Diamino-2,6-dimethoxypyridin, als Blaukuppler Verwendung finden. Neben den genannten Verbindungen sind weiterhin die Kupplersubstanzen 4,6-Dichlorresorcin, 2-Methylresorcin, 2,4-Dihydroxyanisol, 5-Amino-2-methyl-phenol, 1-Naphtol, 4-Amino-2-hydroxyphenoxyethanol und 4-Hydroxyindol als Nuanceure von Interesse.

Das beschriebene Farbsystem auf Basis der genannten Entwickler- und Kupplersubstanzen hat sich zwar in der Praxis bewährt, es hat jedoch einige Nachteile, die beseitigt werden müssen. So entstehen beispielsweise bei der oxidativen Haarfärbung mit den Entwicklersubstanzen 2,5-Diaminotoluol oder 1,4-Diaminobenzol in Kombination mit den Kupplersubstanzen Resorcin oder 4-Chlorresorcin Haarfärbungen, welche nicht völlig natürlich aussehen, sondern rostig wirkende Reflexe zeigen. Bekanntlich ist aber besonders bei Frauen mit stark ergrauten Haaren der Wunsch nach einer natürlichen Haarfärbung besonders ausgeprägt. Diesem Wunsch kann mit den bekannten Oxidationshaarfärbemitteln nicht völlig zufriedenstellend entsprochen werden.

Es ist schon mehrfach versucht worden, die geschilderten Nachteile der Entwicklersubstanzen 2,5-Diaminotoluol und 1,4-Diaminobenzol zu beseitigen, indem die Hydrogenatome an den Aminogruppen dieser Moleküle durch bestimmte Reste, wie zum Beispiel Carbamoylmethyl oder Hydroxyethyl, substituiert wurden. Eine solche Substitution führte aber in der Regel zu Entwicklersubstanzen mit verschlechterten Färbeeigenschaften. Diese Verschlechterung zeigt sich in einem ungenügenden Farbausgleich und/oder dem völligen Fehlen der Bildung natürlicher Nuancen bei der Anwendung der üblichen Entwickler in Verbindung mit Resorcin, 4-Chlorresorcin, 4,6-Dichlorresorcin, 4-Hydroxy-1,2-methylendioxybenzol und 4-Amino-1,2-methylendioxybenzol als Kupplersubstanzen.

Es wurde nun überraschenderweise gefunden, daß bei Ersatz eines Hydrogenatomes am Benzolkern des 1,4-Diaminobenzols durch einen Alkylsulfonylethylrest die vorstehend angeführten Nachteile nicht bestehen. Diese neuen Entwicklersubstanzen ergeben in Kombination mit den Kupplersubstanzen Resorcin, 4-Chlor-resorcin, 4,6-Dichlorresorcin, 4-Hydroxy-1,2-methylen-dioxybenzol und 4-Amino-1,2-methylendioxybenzol sehr natürlich aussehende blonde bis braune Farbtone.

Gegenstand der vorliegenden Patentanmeldung sind daher 2-Alkylsulfonylethyl-1,4-diaminobenzole der Formel I

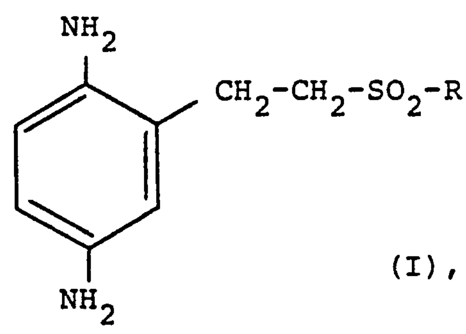

(I),

worin R die Bedeutung $CH_3$ oder $C_2H_5$ hat, und deren Salze sowie Mittel zur oxidativen Färbung von menschlichen Haaren auf der Basis einer Entwicklersubstanz-Kupplersubstanz-Kombination, welche als Entwicklersubstanz eine Verbindung der Formel I oder deren Salze enthalten.

Die Herstellung der neuen 2-Alkylsulfonylethyl-1,4-diaminobenzole erfolgt zweckmäßigerweise in acht Synthesestufen gemäß dem nachstehenden Reaktionsschema, wobei von der in technischen Mengen zur Verfügung stehenden 2-Nitro-phenylessigsäure (II) ausgegangen wird. Diese wird mit Natriumborhydrid in Tetrahydrofuran zur ebenfalls bekannten Verbindung 2-Nitrophenylethanol (III) reduziert. Das 2-Nitrophenylethanol wird sodann mit Bromwasserstoff in konzentrierter Schwefelsäure zur bekannten

Verbindung 2-Nitrophenylethylbromid (IV) bromiert. Die weitere Synthese erfolgt, indem man das 2-Nitrophenylethylbromid mit einem Alkylmercaptan zur neuen Verbindung 2-Alkyl-thioethyl-nitrobenzol (V) umsetzt, das 2-Alkylthioethyl-nitrobenzol mit Hydrogenperoxid zu 2-Alkylsulfonylethyl-nitrobenzol (VI) oxidiert, das 2-Alkylsulfonylethyl-nitrobenzol in Gegenwart von Essigsäureanhydrid und Palladium auf Aktivkohle zu 2-Alkylsulfonylethyl-acetanilid (VII) hydriert, das 2-Alkylsulfonylethyl-acetanilid mit $HNO_3/H_2SO_4$ zu 2-Alkylsulfonylethyl-4-nitroacetanilid (VIII) nitriert, das 2-Alkylsulfonylethyl-4-nitroacetanilid durch Umsetzung mit einer ethanolischen Salzsäurelösung entacetyliert und das erhaltene 2-Alkylsulfonyl-ethyl-4-nitroanilin (IX) schließlich in ethanolischer Lösung durch Hydrieren in Gegenwart von Palladium auf Aktivkohle in das gewünschte 2-Alkylsulfonylethyl-1,4-diaminobenzol (I) überführt.

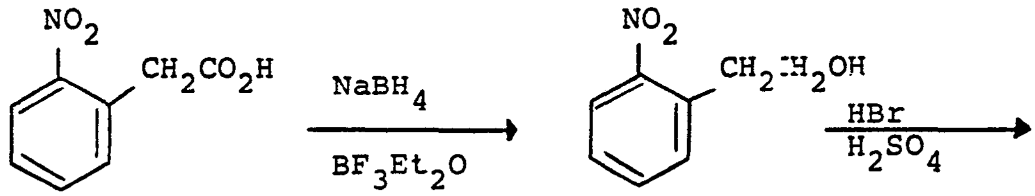


(II) (III)

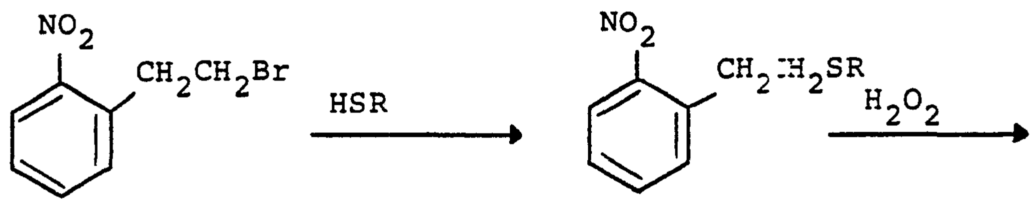


(IV) (V)

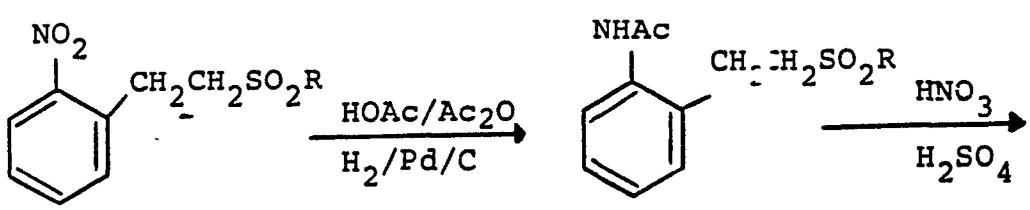


(VI) (VII)

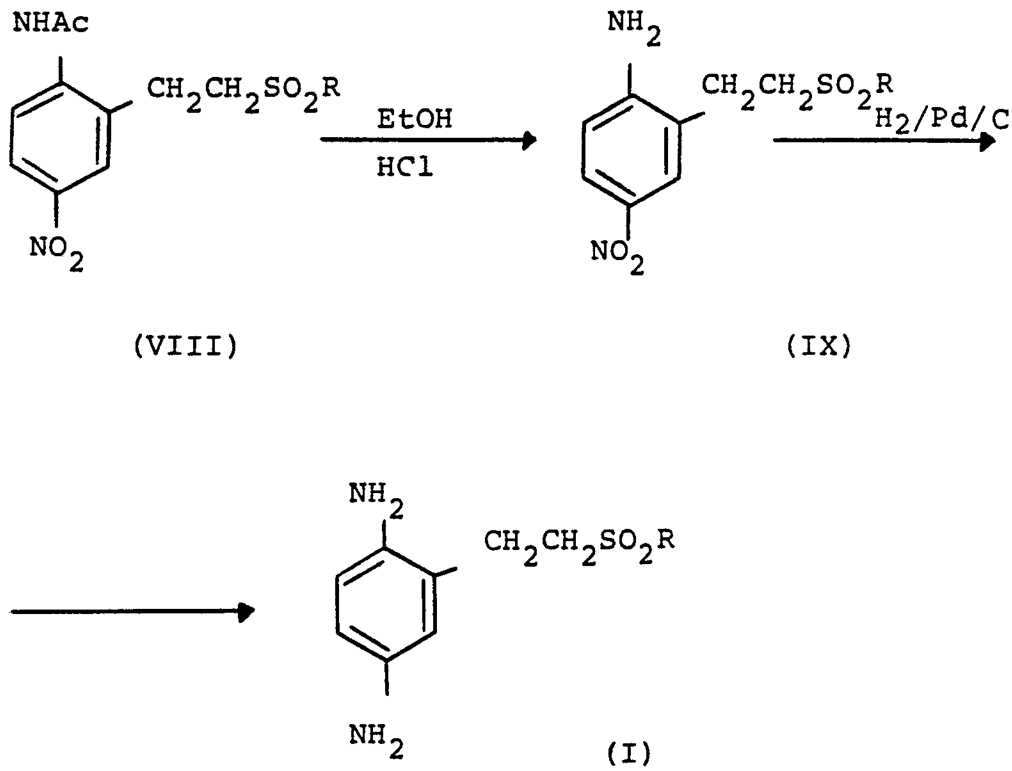


(VIII) (IX)

(I)

Die neuen Entwicklersubstanzen der Formel I sind gut in Wasser löslich und weisen eine ausgezeichnete Lagerbeständigkeit, insbesondere als Bestandteil der hier beschriebenen Haarfärbemittel, auf.

Verwendet man die erfindungsgemäße Entwicklersubstanz 2-Methylsulfonylethyl-1,4-diaminobenzol in Kombination mit den bekannten Kupplersubstanzen Resorcin, 4-Chlor-resorcin und 4,6-Dichlorresorcin, so ist festzustellen, daß in der angegebenen Reihenfolge mit jedem Chloratom, das in das Resorcinmolekül eingeführt wird, die Intensität der erhaltenen Haarfärbung zunimmt. Dementsprechend ist die Verwendung von 2-Methylsulfonyl-1,4-dia-minobenzol zusammen mit 4-Chlorresorcin und 4,6-Dichlor-resorcin bevorzugt. Weiterhin lassen sich auch mit den Kupplersubstanzen 4-Hydroxy-1,2-methylendioxybenzol und 4-amino-1,2-methylendioxybenzol sehr brauchbare natürliche Haarfärbungen erzeugen.

Während die bekannte Entwicklersubstanz 2,5-Diamino-toluol in Kombination mit 4,6-Dichlorresorcin intensive rostige Färbungen ergibt, die für die Färbepraxis ungeeignet sind, liefert 4,6-Dichlorresorcin mit den erfindungsgemäßen Entwicklersubstanzen der Formel I wesentlich natürlichere und daher besser brauchbare Haarfärbungen. Die Färbungen sind zudem auch bei einem vom Haaransatz zu den Haarspitzen hin zunehmenden Schädigungsgrad sehr gleichmäßig.

Eine weitere wichtige positive Eigenschaft der neuen Entwicklersubstanz 2-Methylsulfonylethyl-1,4-diaminobenzol besteht darin, daß sie, sowohl in Abwesenheit des Oxidationsmittels $H_2O_2$ als auch nach der Einwirkung von $H_2O_2$, bei der Prüfung in einer Testanordnung nach Ames nicht mutagen ist.

In den Haarfärbemitteln sollen die erfindungsgemäßen Entwicklersubstanzen, von denen das 2-Methylsulfonyl-ethyl-1,4-diaminobenzol bevorzugt ist, in einer Konzentration von etwa 0,01 bis 4,0 Gew.%, vorzugsweise 0,1 bis 2,5 Gew.%, enthalten sein.

Obwohl die vorteilhaften Eigenschaften der hier beschriebenen neuen Entwicklersubstanzen es nahelegen, diese als alleinige Entwickler zu verwenden, ist es selbstverständlich auch möglich, die neuen Entwicklersubstanzen der Formel I gemeinsam mit bekannten Entwicklersubstanzen, wie z. B. 4-Aminophenol oder 2,5-Diaminotoluol, einzusetzen.

Von den bekannten Kupplersubstanzen kommen als Bestandteil der hier beschriebenen Haarfärbemittel vor allem Resorcin, 4-Chlorresorcin, 4,6-Dichlorresorcin, 2-Methylresorcin, 2-Amino-4-(2'-hydroxyethylamino)-anisol, 2,4-Dihydroxyanisol, 2,4-Dihydroxyphenoxyethanol, 5-Amino-2-methyl-phenol, 2,4-Diaminophenoxyethanol, 4-Amino-2-hydroxyphenoxyethanol, 1-Naphthol, m-Aminophenol, 3-Amino-2-methylphenol, 3-Amino-6-methylphenol, 4-Hydroxy-1,2-methylendioxy-benzol, 4-Amino-1,2-methylendioxybenzol, 4-(2'-Hydroxyethylamino)-1,2-methylendioxybenzol, 2,4-Diaminoanisol und 2,4-Diaminophenetol, 4-Hydroxyindol und 3,5-Diamino-2,6-dimethoxypyridin in Betracht.

Die genannten Kuppler- und Entwicklersubstanzen können in den Haarfärbemitteln jeweils einzeln oder im Gemisch miteinander enthalten sein.

Die Gesamtmenge der in den hier beschriebenen Haarfärbemitteln enthaltenen Entwicklersubstanz-Kupplersubstanz-Kombination soll etwa 0,1 bis 6,0 Gew. %, vorzugsweise 0,5 bis 4,0 Gew. %, betragen.

Die Entwicklerkomponenten werden im allgemeinen in etwa äquimolaren Mengen, bezogen auf die Kupplerkomponenten, eingesetzt. Es ist jedoch nicht nachteilig, wenn die Entwicklerkomponente diesbezüglich in einem gewissen Überschuß oder Unterschuß vorhanden ist. Besonders zur Erzielung matter Nuancen kann es gegebenenfalls günstig sein, die Entwicklerkomponente im Unterschuß zu verwenden.

Weiterhin können die Haarfärbemittel dieser Anmeldung zusätzlich andere Farbkomponenten, beispielsweise 6-Amino-2-methyl-phenol und 6-Amino-3-methylphenol, sowie ferner übliche direktziehende Farbstoffe, zum Beispiel Triphenylmethanfarbstoffe wie Diamond Fuchsine (C.I. 42 510) und Leather Ruby HF (C.I. 42 520), aromatische Nitrofarbstoffe wie 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethylamino)-nitrobenzol und 2-Methylamino-5-bis-(2'-hydroxyethyl)-amino-nitrobenzol, Azofarbstoffe wie Acid Brown 4 (C.I. 14 805) und Dispersionsfarbstoffe, wie beispielsweise 1,4-Diaminoanthrachinon und 1,4,5,8-Tetraaminoanthrachinon, enthalten. Die Haarfärbemittel können diese Farbkomponenten in einer Menge von etwa 0,1 bis 4,0 Gew.% enthalten.

Selbstverständlich können die Kuppler- und Entwicklersubstanzen sowie die anderen Farbkomponenten, sofern es Basen sind auch in Form der physiologisch verträglichen Säureadditionssalze, wie beispielsweise als Hydrochlorid bzw. Sulfat oder - sofern sie aromatische OH-Gruppen besitzen - in Form der Salze mit Basen, zum Beispiel als Alkaliphenolate, eingesetzt werden.

Darüber hinaus können in den Haarfärbemitteln noch weitere übliche kosmetische Zusätze, beispielsweise Antioxidantien wie Ascorbinsäure, Thioglykolsäure oder Natriumsulfit, Parfümöle, Komplexbildner, Netzmittel, Emulgatoren, Verdicker, Pflegestoffe und andere vorhanden sein.

Die Zubereitungsform kann beispielsweise eine Lösung, insbesondere eine wäßrige oder wäßrigalkoholische Lösung, sein. Die besonders bevorzugten Zubereitungsformen sind jedoch eine Creme, ein Gel oder eine Emulsion.

Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol und Isopropanol, oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gew.%.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid, Verwendung finden.

Bei der Anwendung zur oxidativen Färbung von Haaren vermischt man die hier beschriebenen Haarfärbemittel auf der Basis einer Entwicklersubstanz-Kupplersubstanz-Kombination sowie gegebenenfalls anderer üblicher Farbkomponenten kurz vor dem Gebrauch mit einem Oxidationsmittel und trägt eine zur Färbung des Haares ausreichende Menge der Mischung, im allgemeinen etwa 50 bis 150 ml, auf das Haar auf. Als Oxidationsmittel zur Entwicklung der Haarfärbung kömmt hauptsächlich Hydrogenperoxid, beispielsweise als 6 %ige wäßrige Lösung bzw. dessen Additionsverbindungen an Harnstoff, Melamin oder Natriumborat in Betracht. Man läßt das Gemisch bei 15 bis 50°C etwa 10 bis 45 Minuten lang, vorzugsweise 30 Minuten lang, auf das Haar einwirken, spült sodann das Haar mit Wasser aus und trocknet. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Abschließend wird das Haar getrocknet.

Die nachstehenden Beispiele sollen den Gegenstand der Erfindung näher erläutern.

**Herstellungsbeispiel**

**Beispiel 1**

1. Stufe

Herstellung von 2-Nitrophenylethylalkohol (III)

91,0 g (0,5 Mol) 2-Nitrophenylessigsäure werden in 500 ml Tetrahydrofuran gelöst und mit 32,16 g (0,85 Mol) Natriumborhydrid versetzt. Nachdem die Wasserstoffentwicklung abgeklungen ist, werden während 2 Stunden 200 ml Bortrifluordiethyletherat zugetropft. Die Mischung wird weitere 2 Stunden lang bei Raumtemperatur

gerührt. Danach werden 220 ml 2-normaler Salzsäurelösung zugetropft. Die Wasserphase wird viermal mit je 100 ml Diethylether extrahiert. Die Etherphase wird mit 2-normaler Natriumcarbonatlösung und mit Wasser neutral gewaschen und über Magnesiumsulfat getrocknet.

Das beim Abziehen des Ethers zurückbleibende Öl (77 g) wird im Vakuum destilliert. Die bei 115-118° C/5 Pa (Pascal) siedende Fraktion wiegt 66 g. Dies entspricht einer Ausbeute von 78 % der Theorie. Für die Verbindung III ist in der Literatur (Beilstein EII 6 (1944) 452) ein Siedepunkt von 165-167° C/6 Torr (≙ 800 Pa) angegeben.

2. Stufe

Herstellung von 2-Nitrophenylethylbromid (IV)

65 g (0,39 Mol) 2-Nitrophenylethanol (III) werden mit 200 ml Bromwasserstoffsäure (48 %-ig) und 45 ml konzentrierter Schwefelsäure 18 Stunden lang auf 110° C erwärmt. Danach werden 100 ml Wasser zugesetzt. Die Mischung wird mit Methylenchlorid extrahiert; die organischen Extrakte werden mit 2-normaler Natriumcarbonatlösung und Wasser neutral gewaschen und über Magnesiumsulfat getrocknet.

Das nach dem Abziehen des Methylenchlorids zurückbleibende Öl (88 g) wird mit 50 ml Ethanol gelöst und auf 0° C abgekühlt. Das gewünschte Produkt kristallisiert nach Anreiben mit einem Glasstab aus.

Ausbeute: 71 g (80 % der Theorie)

Schmelzpunkt: 35 bis 36° C (Literatur: E.L. Foreman and S.M. McElvain, J. Am. Chem. Soc. (1940), 1435 - 1338: 36-38° C)

3. Stufe

Herstellung von 2-Methylthioethyl-nitrobenzol

16,0 g (0,4 Mol) Natriumhydroxid werden in 120 ml Wasser und 230 ml Ethanol gelöst. In diese Lösung werden 37 g (0,78 Mol) Methylmercaptan eingeleitet. Die Mischung wird auf 90° C erwärmt und mit 71 g (0,31 Mol) 2-Nitrophenylethylbromid (IV), gelöst in 450 ml Ethanol, tropfenweise während zwei Stunden versetzt.

Nachdem die Lösung anschließend eine Stunde unter Rückfluß gekocht worden ist, wird das Ethanol abdestilliert. Der Rückstand wird mit Wasser versetzt und mit Methylenchlorid extrahiert. Das aus der Methylenchloridphase erhaltene Öl (56 g) kann im Vakuum destilliert werden. Es hat einen Siedepunkt von 105 - 106° C bei 3 Pa.

4. Stufe

Herstellung von 2-Methylsulfonylethyl-nitrobenzol

29,0 g (0,15 Mol) 2-Methylthioethyl-nitrobenzol werden in 150 ml Eisessig mit 70 ml Hydrogenperoxid (30 %ige wäßrige Lösung), tropfenweise versetzt. Die Temperatur der Mischung wird durch Kühlung bei 40° C gehalten. Danach wird die Mischung eine Stunde lang auf 100° C erwärmt. Der abgekühlten Lösung werden 300 ml Wasser zugesetzt, wodurch das gewünschte Produkt kristallin ausgefällt wird. Es wird abgesaugt und aus Ethanol umkristallisiert.

Ausbeute: 29 g (85 % der Theorie)

Schmelzpunkt: 100 - 101° C

| | | | | |
|---|---|---|---|---|
| CHN-Analyse | $C_9H_{11}NO_4S$ | %C | %H | %N |
| | berechnet: | 47,15 | 4,84 | 6,11 |
| | gefunden: | 47,14 | 4,83 | 6,10 |

5. Stufe

Herstellung von 2-Methylsulfonylethyl-acetanilid

8,0 g (35 mMol) 2-Methylsulfonylethyl-nitrobenzol werden in einem Gemisch aus 160 ml Eisessig und 160 ml Essigsäureanhydrid unter Zusatz von 0,4 g Palladium auf Aktivkohle (10 % Pd) hydriert. Nach drei Stunden wird der Katalysator abfiltriert und die überschüssigen Lösungsmittel werden unter vermindertem Druck vollständig abdestilliert.

Ausbeute (roh): 10 g

Zur Analyse wurde eine Probe aus Ethänol umkristallisiert.

Schmelzpunkt. 142 - 143° C

| | | | | |
|---|---|---|---|---|
| CHN-Analyse: | $C_{11}H_{15}NO_3S$ | %C | %H | %N |
| | berechnet: | 54,75 | 6,27 | 5,80 |
| | gefunden: | 54,72 | 6,28 | 5,74 |

6. und 7. Stufe

Herstellung von 2-Methylsulfonylethyl-4-nitroanilin

Das Rohprodukt aus der 5. Stufe wird in 100 ml konzentrierter Schwefelsäure gelöst, auf 0° C abgekühlt und mit 3,2 ml Salpetersäure (Dichte: 1,52 kg/m$^3$) bei 0° C nitriert. Die Mischung wird während einer Stunde auf Raumtemperatur erwärmt und dann auf Eis gegossen. Das ausgeschiedene Produkt wird abgesaugt, mit Wasser gewaschen und getrocknet.

Das Produkt wird mit 150 ml Ethanol und 50 ml Salzsäure (Dichte 1,16 kg/m$^3$) eine halbe Stunde lang auf 100° C erwärmt. Danach werden 100 ml Wasser zugesetzt. Das in gelben Nadeln kristallisierende Produkt wird abgesaugt und aus 1,3 l Ethanol umkristallisiert.

Ausbeute: 4,0 g (47 % der Theorie bezogen auf das eingesetzte 2-Methylthioethyl-4-nitrobenzol)

Schmelzpunkt: 197 - 198° C

| CHN-Analyse: | $C_9H_{12}N_2O_4S$ | %C | %H | %N |
|---|---|---|---|---|
| | berechnet: | 44,25 | 4,95 | 11,47 |
| | gefunden: | 44,26 | 4,95 | 11,53 |

8. Stufe

Herstellung von 2-Methylsulfonylethyl-1,4-diamino-benzol-sulfat

2,0 g 2-Methylsulfonylethyl-4-nitroanilin werden in 750 ml Ethanol mit 0,35 g Palladium auf Aktivkohle (10 % Pd) hydriert. Nach vier Stunden wird vom Katalysator abfiltriere. Das Filtrat wird mit 20 ml 2-normaler Schwefelsäure versetzt. Das ausgefallene Produkt wird abgesaugt, mit Ethanol gewaschen und getrocknet.

Ausbeute: Es werden 2,5 g (100 % der Theorie) eines weißen Pulvers erhalten.

| CHN-Analyse: | $C_9H_{14}O_2S \cdot H_2SO_4$ | %C | %H | %N |
|---|---|---|---|---|
| | berechnet: | 34,61 | 5,16 | 8,97 |
| | gefunden: | 34,06 | 5,21 | 8,66 |

Titration: Einwaage 31,0 mg Auslauf an 0,01-normaler Natronlauge:
19,9 ml (berechnet)
20,3 ml (gefunden)

Die freie Base kann aus einem Hydrieransatz durch Einengen der ethanolischen Lösung bis zur beginnenden Kristallisation gewonnen werden.

Schmelzpunkt: Die farblosen Blättchen des Reaktionsproduktes schmelzen bei 147 bis 149° C

| CHN-Analyse: | $C_9H_{14}N_2O_2S$ | %C | %H | %N |
|---|---|---|---|---|
| | berechnet: | 50,45 | 6,58 | 13,07 |
| | gefunden: | 50,48 | 6,61 | 13,00 |

Die 1,4-Stellung der Aminogruppen in der synthetisierten Verbindung ergibt sich aus der Tatsache, daß sie bei Ausfärbungen mit den üblichen Kupplern die für p-Diamine charakteristischen Farbreaktionen zeigt.

**Beispiele für Haarfärbemittel**

**Beispiel 2** Haarfärbemittel in Gelform

| | |
|---|---|
| 0,5 g | 2-Methylsulfonylethyl-1,4-diaminobenzol-sulfat |
| 0,5 g | 4,6-Dichlorresorcin |
| 0,3 g | Ascorbinsäure |
| 1,0 g | Hydroxyethylcellulose, hochviskos |
| 5,0 g | Natriumlaurylalkoholdiglykolethersulfat (28 %ige wäßrige Lösung) |
| 10,0 g | Ammoniak, 25-%ig |
| 82,7 g | Wasser |
| ------ | |
| 100,0 g | |

Man vermischt kurz vor dem Gebrauch 50 g dieses Haarfärbemittels mit 50 ml Hydrogenperoxidlösung (6 %ig) und läßt das Gemisch 30 Minuten lang bei 40° C auf blondierte menschliche Haare einwirken. Danach wird mit Wasser gespült und getrocknet. Das Haar hat eine natürliche dunkelblonde Färbung angenommen.

**Beispiel 3** Haarfärbemittel in Gelform

| | |
|---|---|
| 1,0 g | 2-Methylsulfonylethyl-1,4-diaminobenzol-sulfat |
| 0,9 g | 4-Amino-1,2-methylendioxybenzol |
| 0,1 g | m-Aminophenol |
| 0,3 g | Ascorbinsäure |
| 15,0 g | Ölsäure |
| 7,0 g | Isopropanol |
| 10,0 g | Ammoniak, 25 %ig |
| 65,7 g | Wasser |
| ----- | |
| 100,0 g | |

50 g dieses Haarfärbemittels werden kurz vor dem Gebrauch mit 50 ml Hydrogenperoxidlösung (6 %ig) vermischt und das Gemisch anschließend auf ergrautes, chemisch nicht geschädigtes Haar aufgetragen. Nach einer Einwirkungszeit von 30 Minuten bei 40°C wird mit Wasser gespült und getrocknet. Das Haar ist mittelbraun gefärbt.

**Beispiel 4** Haarfärbemittel in Cremeform

| | |
|---|---|
| 3,0 g | Methylsulfonylethyl-1,4-diaminobenzol-sulfat |
| 1,2 g | 4-Chlorresorcin |
| 0,6 g | 2-Amino-4-(2'-hydroxyethylamino)-anisol-sulfat |
| 0,3 g | m-Aminophenol |
| 0,3 g | Natriumsulfit, wasserfrei |
| 15,0 g | Cetylalkohol |
| 3,5 g | Natriumlaurylalkoholdiglykolethersulfat (28 %ige wäßrige Lösung) |
| 8,0 g | Ammoniak, 25 %ig |
| 68,1 g | Wasser |
| ————— | |
| 100,0 g | |

50 g dieses Haarfärbemittels werden kurz vor dem Gebrauch mit 50 ml Hydrogenperoxidlösung (6 %ig) vermischt und das Gemisch anschließend auf blonde menschliche Haare aufgetragen. Nach einer Einwirkungszeit von 30 Minuten bei 40°C wird mit Wasser gespült und getrocknet. Das Haar ist in einem intensiven, natürlichen Schwarz gefärbt.

Alle in der vorliegenden Anmeldung angegebenen Prozentzahlen stellen Gewichtsprozente dar.

## Patentansprüche

1. 2-Alkylsulfonylethyl-1,4-diaminobenzol der Formel I

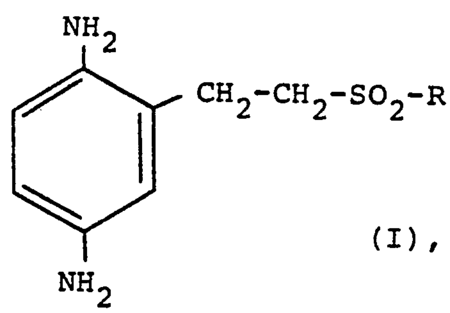

(I),

worin R die Bedeutung $CH_3$ oder $C_2H_5$ hat und dessen Salze.

2. 2-Methylsulfonylethyl-1,4-diaminobenzol und dessen Salze.

3. Verfahren zur Herstellung der Verbindung nach Anspruch 1, dadurch gekennzeichnet, daß man 2-Nitrophenylethylbromid mit einem Alkylmercaptan zu 2-Alkylthioethyl-nitrobenzol umsetzt, das 2-Alkyl-thioethylnitrobenzol mit Hydrogenperoxid zu 2-Alkylsulfonylethyl-nitrobenzol oxidiert, das 2-Alkylsulfonylethylnitrobenzol in Gegenwart von Essigsäureanhydrid zu 2-Alkylsulfonylethyl-acetani-lid hydriert, das 2-Alkylsulfonylethyl-acetanilid nitriert, das entstandene 2-Alkylsulfonylethyl-4-nitroacetanilid durch Umsetzung mit einer ethanolischen Salzsäurelösung entacetyliert und das erhaltene 2-Alkylsulfonylethyl-4-nitroanilin schließlich durch Hydrierung zu 2-Alkylsulfonylethyl-1,4-diaminobenzol umsetzt.

4. Mittel zur oxidativen Färbung von menschlichen Haaren auf der Basis einer Entwicklersubstanz-Kupplersubstanz-Kombination, dadurch gekennzeichnet, daß es als Entwicklersubstanz ein 2-Alkylsulfonylethyl-1,4-diaminobenzol der Formel I

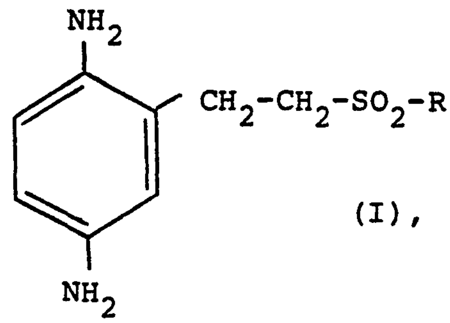

(I),

worin R die Bedeutung $CH_3$ oder $C_2H_5$ hat, oder dessen Salze enthält.

5. Mittel nach Anspruch 4, dadurch gekennzeichnet, daß es die Entwicklersubstanz der Formel I in einer Menge von 0,01 bis 4,0 Gew.%, vorzugsweise 0,1 bis 2,5 Gew.%, enthält.

6. Mittel nach Anspruch 4 und 5, dadurch gekennzeichnet, daß es als Entwicklersubstanz 2-Methylsulfonylethyl-1,4-diaminobenzol oder dessen Salze enthält.

7. Mittel nach den Ansprüchen 4 bis 6, dadurch gekennzeichnet, daß es mindestens eine der Kupplersubstanzen Resorcin, 2-Methylresorcin, 4-Chlorresorcin, 4,6-Dichlorresorcin, 4-Hydroxy-1,2-methylendioxybenzol, 4-Amino-1,2-methylendioxybenzol, 4-(2'-Hydroxyethylamino)-1,2-methylendioxybenzol, 2,4-Dihydroxyanisol, 2,4-Dihydroxyphenoxyethanol, 4-Amino-2-hydroxyphenoxyethanol, m-Aminophenol, 5-Amino-2-methyl-phenol, 1-Naphthol, 4-Hydroxyindol, 2,4-Diaminoanisol, 2,4-Diaminophenetol, 2-Amino-4-(2'-hydroxyethylamino)-anisol, 2,4-Diaminophenoxyethanol und 3,5-Diamino-2,6-dimethoxypyridin enthält.

8. Mittel nach Anspruch 4 bis 7, dadurch gekennzeichnet, daß die Gesamtmenge der Kupplersubstanz-Entwicklersubstanz-Kombination etwa 0,1 bis 6,0 Gew.%, vorzugsweise 0,5 bis 4,0 Gew.%, beträgt.

9. Mittel nach Anspruch 4 bis 8, dadurch gekennzeichnet, daß es eine Farbkomponente enthält, welche ausgewählt ist aus 6-Amino-2-methylphenol, 6-Amino-3-methylphenol, Diamond Fuchsine (C.I. 42 510), Leather Ruby HF (C.I. 42 520), 2-Nitro-1,4-diaminobenzol, 2-Amino-4-nitrophenol, 2-Amino-4,6-dinitrophenol, 2-Amino-5-nitrophenol, 2-Amino-5-(2'-hydroxyethylamino)-nitrobenzol, 2-Methylamino-5-bis-(2'-hydroxyethyl)-amino-nitrobenzol, Acid Brown 4 (C.I. 14 805), 1,4-Diaminoanthrachinon und 1,4,5,8-Tetraaminoanthrachinon.

10. Mittel nach Anspruch 4 bis 9, dadurch gekennzeichnet, daß es Antioxidantien, vorzugsweise Ascorbinsäure, Natriumsulfit oder Thioglykolsäure, enthält.

**Claims**

1. 2-alkyl sulphonyl ethyl-1,4-diamino benzene to formula I

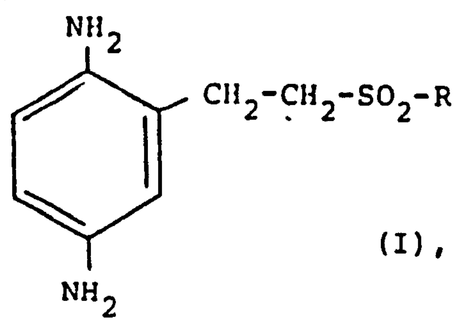

(I),

in which R denotes $CH_3$ or $C_2H_5$ and the salts thereof.

2. 2-methyl sulphonyl ethyl-1,4-diamino benzene and the salts thereof.

3. A method of producing the compound according to Claim 1, characterised in that 2-nitro-phenyl ethyl bromide is reacted with an alkyl mercaptan to produce 2-alkyl thioethyl-nitro benzene, the 2-alkyl thioethyl-nitro benzene is oxidised with hydrogen peroxide to produce 2-alkyl sulphonyl ethyl-nitro benzene, the 2-alkyl sulphonyl ethyl-nitro benzene is hydrated in the presence of acetic acid anhydride to produce 2-alkyl sulphonyl ethylacetanilide, the 2-alkyl sulphonyl ethyl acetanilide is nitrated, the resultant 2-alkyl sulphonyl ethyl-4-nitro-acetanilide is deacetylated by reaction with an ethanolic hydrochloric acid solution and the 2-alkyl sulphonyl ethyl-4-nitro aniline obtained is finally reacted by hydration to produce 2-alkyl sulphonyl ethyl-1,4-diamino benzene.

4. Agent for the oxidative colouring of human hair, based on a developer substance-coupler substance combination, characterised in that as a developer substance it contains a 2-alkyl sulphonyl ethyl-1,4-diamino benzene to formula I

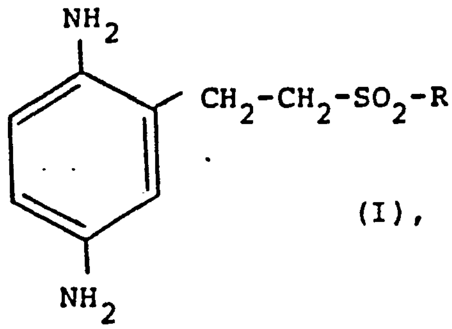

(I),

in which R denotes $CH_3$ or $C_2H_5$ or the salts thereof.

5. Agent according to Claim 4, characterised in that it contains the developer substance to formula I in a quantity of 0.01 to 4 % by weight and preferably of 0.1 to 2.5 % by weight.

6. Agent according to Claim 4 and 5, characterised in that as a developer substance it contains 2-methyl sulphonyl ethyl-1,4-diamino benzene or the salts thereof.

7. Agent according to Claims 4 to 6, characterised in that it contains at least one of the coupler substances resorcin, 2-methyl resorcin, 4-chloro resorcin, 4,6-dichloro resorcin, 4-hydroxy-1,2-methylene dioxy benzene, 4-amino-1,2-methylene dioxy benzene, 4-(2'-hydroxy ethyl amino)-1,2-methylene dioxy benzene, 2,4-dihydroxy anisol, 2,4-dihydroxy phenoxy ethanol, 4-amino-2-hydroxy phenoxy ethanol, m-amino phenol, 5-amino-2-methyl phenol, naphthol, 4-hydroxy indol, 2,4-diamino anisol, 2,4-diamino phenetol, 2-amino-4-(2'-hydroxy ethyl amino)-anisol, 2,4-diamino phenoxy ethanol and 3,5-diamino-2,6-dimethoxy pyridin.

8. Agent according to Claim 4 to 7, characterised in that the total quantity of coupler substance-developer substance combination amounts to about 0.1 to 6 % by weight and preferably 0.5 to 4 % by weight.

9. Agent according to Claim 4 to 8, characterised in that it contains a colour component which is chosen from 6-amino-2-methyl-phenol, 6-amino-3-methyl phenol, Diamond Fuchsine (C.I. 42 510), Leather Ruby HF (C.I. 42 520), 2-nitro 4,6-diamino benzene, 2-amino-4-nitro phenol, 2-amino-4,6-dinitro phenol, 2-amino-5-nitro phenol, 2-amino-5-(2'-hydroxy ethyl amino)-nitro benzene, 2-methyl amino-5-bis-(2'-hydroxy ethyl)-amino-nitro benzene, Acid Brown 4 (C.I. 14 805), 1,4-diamino anthraquinone and 1,4,5,8-tetra amino anthraquinone.

10. Agent according to Claim 4 to 9, characterised in that it contains antioxidants, preferably ascorbic acid, sodium sulphite or thioglycolic acid.

**Revendications**

1. 2-alcoylsulfonyléthyl-1,4-diaminobenzène répondant à la formule I

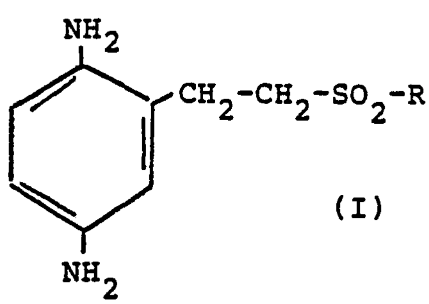

(I)

dans laquelle R représente $CH_3$ ou $C_2H_5$, et ses sels.

2. 2-méthylsulfonyléthyl-1,4-diaminobenzène et ses sels.

3. Procédé de préparation du composé selon la revendication 1, caractérisé en ce qu'on transforme du bromure de 2-nitrophényléthyle en 2-alcoylthioéthyl-nitrobenzène par réaction sur un alcolyl-mercaptan, on oxyde le 2-alcoylthioéthyl-nitrobenzène en 2-alcoylsulfonyléthyl-nitrobenzène avec de l'eau oxygénée, on hydrogène le 2-alcoylsulfonyléthyl-nitrobenzène en 2-alcoylsulfonyléthyl-acétanilide en présence d'anhydride acétique, on nitre le 2-alcoylsulfonyléthyl-acétanilide, on désacétyle le 2-alcoylsulfonyléthyl-4-nitro-acétanilide formé par réaction sur une solution d'acide chlorhydrique dans l'éthanol et, enfin, on transforme la 2-alcoylsulfonyléthyl-4-nitro-aniline obtenue en 2-alcoylsulfonyléthyl-1,4-diaminobenzène par hydrogénation.

4. Produit de coloration par oxydation de cheveux humains à base d'une combinaison développeur-copulateur, caractarisé en ce qu'il renferme, comme développeur, un 2-alcoylsulfonyléthyl-1,4-diaminobenzène répondant à la formule I

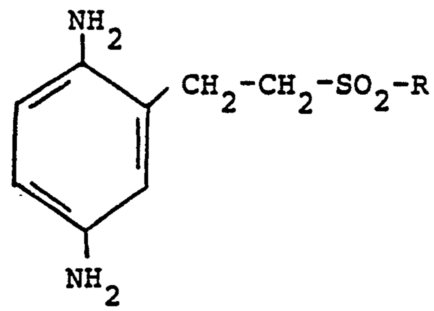

(I)

dans laquelle R représente $CH_3$ ou $C_2H_5$, ou ses sels.

5. Produit selon la revendication 4, caractérisé en ce qu'il renferme le développeur répondant à la formule I dans une proportion de 0,01 à 4,0 % en poids, de préférence de 0,1 à 2,5 % en poids.

6. Produit selon les revendications 4 et 5, caractérisé en ce qu'il renferme, comme développeur, du 2-méthylsulfonyléthyl-1,4-diaminobenzène ou ses sels.

7. Produit selon les revendications 4 à 6, caractérisé en ce qu'il renferme l'un au moins des copulateurs suivants: la resorcine, la 2-méthyl-résorcine, la 4-chloro-résorcine, la 4,6-dichloro-résorcine, le 4-hydroxy-1,2-éthylène-dioxybenzène, le 4-amino-1,2-méthylène-dioxybenzène, le 4-(2'-hydroxyéthylamino)-1,2-méthylène-dioxybenzène, le 2,4-dihydroxy-anisole, le 2,4-dihydroxyphénoxy-éthanol, le 4-amino-2-hydroxyphénoxy-éthanol, le m-amino-phénol, le 5-amino-2-méthyl-phénol, le 1-naphtol, le 4-hydroxy-indole, le 2,4-diamino-anisole, le 2,4-diaminophénétol, le 2-amino-4-(2'-hydroxyéthylamino)-anisole, le 2,4-diaminophénoxy-éthanol et la 3,5-diamino-2,6-diméthoxy-pyridine.

8. Produit selon les revendications 4 à 7, caractérisé en ce que la proportion totale de la combinaison copulateur-développeur est d'environ 0,1 à 6,0 % en poids, de préférence de 0,5 à 4,0 % en poids.

9. Produit selon les revendications 4 à 8, caractérisé en ce qu'il renferme un constituant colorant choisi parmi le 6-amino-2-méthyl-phénol, le 6-amino-3-méthyl-phénol, "Diamond Fuchsine" (I.C.42 510), "Leather Ruby HF" (I.C. 42 520), le 2-nitro-1,4-diaminobenzène, le 2-amino-4-nitrophénol, le 2-amino-4,6-dinitrophénol, le 2-amino-5-nitrophénol, le 2-amino-5-(2'-hydroxyéthylamino)-nitrobenzène, le 2-méthylamino-5-bis-(2'-hydroxyéthyl)-amino-nitrobenzène, "Acid Brown 4" (I.C. 14 805), la 1,4-diamino-anthraquinone et la 1,4,5,8-tétra-amino-anthraquinone.

10. Produit selon les revendications 4 à 9, caractérisé en ce qu'il renferme des anti-oxydants, de préférence de l'acide ascorbique, du sulfite de sodium ou de l'acide thioglycolique.